(19) **Europäisches Patentamt / European Patent Office / Office européen des brevets**

(11) **EP 4 541 384 A1**

(12) **EUROPEAN PATENT APPLICATION**
published in accordance with Art. 153(4) EPC

(43) Date of publication:
23.04.2025 Bulletin 2025/17

(21) Application number: 23824226.7

(22) Date of filing: 14.06.2023

(51) International Patent Classification (IPC):
$A61L\ 27/48^{(2006.01)}$ $\quad A61L\ 27/26^{(2006.01)}$
$A61L\ 27/18^{(2006.01)}$ $\quad A61L\ 27/20^{(2006.01)}$
$A61L\ 27/36^{(2006.01)}$ $\quad A61L\ 27/60^{(2006.01)}$

(52) Cooperative Patent Classification (CPC):
A61L 27/18; A61L 27/20; A61L 27/26; A61L 27/36;
A61L 27/48; A61L 27/60

(86) International application number:
PCT/KR2023/008196

(87) International publication number:
WO 2023/244012 (21.12.2023 Gazette 2023/51)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 14.06.2022 KR 20220072131

(71) Applicant: Vaim Co. Ltd.
Okcheon-gun, Chungcheongbuk-do 29055 (KR)

(72) Inventor: KIM, Gun Poong
Okcheon-gun, Chungcheongbuk-do 29055 (KR)

(74) Representative: Dehns
10 Old Bailey
London EC4M 7NG (GB)

(54) **COMPOSITE, METHOD FOR MANUFACTURING SAME, AND FILLER COMPOSITION FOR MOLDING, USING SAME**

(57) The present invention relates to a composite, a method for manufacturing same, and a filler composition for molding, using same, the composite comprising: bio-degradable particles the particle-size distribution (PSD) of which is controlled; and a water-soluble polymer. The composite of the present invention may have an excellent long-term storage property and distribution property.

[Fig. 1]

## Description

### Technical Field

The present invention relates to a composite of two or more components, to a process for preparing the same, and to a filler composition for plastic surgery (e.g., a filler composition for skin plastic surgery) using the same.

### Background Art

Plastic surgery is being performed to inject filler compositions subcutaneously or into tissues for purposes such as correcting human body functions or cosmetic purposes. Such filler compositions are preferably safe for the human body and biocompatible and biodegradable.

Such filler compositions contain natural polymers such as collagen, gelatin, hyaluronic acid, and dextran, or synthetic polymers such as polylactic acid, polyglutamic acid, polycaprolactone, and polyacrylamide.

However, since the natural polymers or the synthetic polymers begin to decompose after a certain period of time, the shelf life of the filler compositions containing them is limited. In addition, most filler compositions are administered using a syringe. In such an event, if the viscosity of a filler composition is too high or the dispersibility is poor, it may be difficult to inject the filler composition with a thin needle, and the force (injection force) applied to the syringe must increase, causing discomfort and fatigue to the operator.

[Prior art documents]

[Patent documents]

(Patent Document 1) Korean Laid-open Patent Publication No. 2017-0123099 (November 7, 2017)

### Disclosure of Invention

### Technical Problem

The present invention aims to provide a composite that has excellent long-term storage and dispersibility and can improve the operating environment of a filler composition for plastic surgery, and a process for preparing the same.

In addition, the present invention aims to provide a filler composition for plastic surgery that can be carried out relatively easily.

### Solution to Problem

According to an embodiment of the present invention to accomplish the above object, there is provided a composite, which comprises biodegradable particles; and a water-soluble polymer, wherein the particle size distribution (PSD) of the biodegradable particles according to the following Equation 1 is 1.0 to 2.5:

$$[\text{Equation 1}]$$

$$PSD = (Dv\,(90) - Dv\,(10))/Dv\,(50)$$

In Equation 1, Dv (10) is the size at which the biodegradable particle distribution is within 10%, Dv (50) is the size at which the biodegradable particle distribution is within 50%, and Dv (90) is the size at which the biodegradable particle distribution is within 90%.

According to another embodiment of the present invention, there is provided a process for preparing a composite, which comprises (1) dissolving a biodegradable raw material in a first solvent to prepare a biodegradable solution; (2) spraying the biodegradable solution into a second solvent having a lower freezing point than that of the first solvent to form biodegradable particles; (3) sorting the biodegradable particles by size; (4) adding the biodegradable particles sorted by size to a water-soluble polymer solution to prepare a mixed solution; (5) filling the mixed solution in a container; and (6) freeze-drying the mixed solution filled in the container to form a composite, wherein the particle size distribution (PSD) of the biodegradable particles contained in the composite according to the above Equation 1 is 1.0 to 2.5.

According to another embodiment of the present invention, there is provided a filler composition for plastic surgery in which the composite is dispersed.

**Advantageous Effects of Invention**

Since the composite according to the present invention comprises biodegradable particles whose particle size distribution is controlled to a specific range, it can be dispersed quickly and uniformly in a solvent, and the decomposition of the biodegradable particles and/or water-soluble polymer contained in the composite can be minimized even when it is stored for a long period of time. Accordingly, when the composite according to the present invention is used as a material for a filler composition for plastic surgery, it is possible to extend the shelf life of the material and improve the convenience of storage and handling.

In addition, since the filler composition for plastic surgery according to the present invention is one in which a composite with excellent dispersibility is dispersed, the treatment can be easily performed even if the operator (the person performing the treatment) applies a relatively small force to the syringe.

**Brief Description of the Drawings**

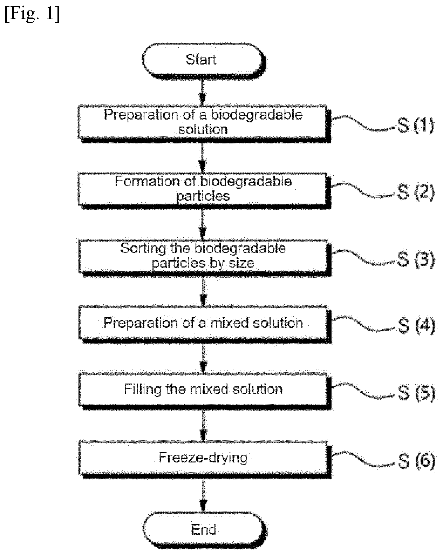

Fig. 1 schematically shows the preparation process of a composite according to an embodiment of the present invention.

Fig. 2 is photographs showing the formulations of the composites according to Example 1, Example 5, and Comparative Example 1 in Test Example 1.

Fig. 3 is an image showing the cross-section of the composite according to Example 1 in Test Example 6 using a scanning electron microscope.

Fig. 4 is an image showing the cross-section of the composite according to Example 5 in Test Example 6 using a scanning electron microscope.

**Best Mode for Carrying out the Invention**

Hereinafter, the present invention will be described in detail. Here, the present invention is not limited to those described below. Rather, it can be modified into various forms as long as the gist of the invention is not altered.

In the present specification, the term "comprising" is intended to specify a particular characteristic, region, step, process, element, and/or component. It does not exclude the presence or addition of any other characteristic, region, step, process, element and/or component, unless specifically stated to the contrary.

In the present specification, in the case where an element is mentioned to be connected or combined, it means all of the cases where one element is directly, or indirectly through another element, connected or combined with another element.

In the present specification, a singular expression is understood to encompass a singular or plural expression, interpreted in context, unless otherwise specified.

All numbers and expressions related to the quantities of components, reaction conditions, and the like used herein may be modified by the term "about" unless otherwise indicated.

Throughout the description of the embodiments, the terms first, second, and so on are used to describe various components. But the components should not be limited by the terms. The terms are used only for the purpose of distinguishing one component from another.

**Composite**

The composite according to the present invention may be a composite of materials having biocompatibility and/or biodegradability. Specifically, the composite according to the present invention comprises biodegradable particles whose particle size distribution (PSD) is controlled to a specific range, which will be described as follows.

Biodegradable particles

The biodegradable particles contained in the composite according to the present invention function to restore or replace damaged or aged human tissue (e.g., skin tissue).

The biodegradable particles have a particle size distribution (PSD) of 1.0 to 2.5 according to the following Equation 1. Specifically, the particle size distribution of the biodegradable particles according to the following Equation 1 may be 1.0 to 2.3, 1.01 to 2.0, 1.02 to 1.9, 1.03 to 1.95, 1.04 to 1.9, 1.05 to 1.8, 1.05 to 1.6, 1.05 to 1.5, 1.08 to 1.4, 1.09 to 1.3, or 1.1 to 1.3, but it is not limited thereto. As the particle size distribution of the biodegradable particles is within the above range, the composite comprising the same may have excellent dispersibility in a solvent. In particular, when the composite according to the present invention is used as a raw material for a filler composition for plastic surgery, the filler composition for plastic surgery can be prepared in a short period of time by virtue of the excellent dispersibility of the composite. In addition, since the filler composition for plastic surgery is prepared using the composite in which the biodegradable particles with a particle size capable of efficiently restoring or replacing human tissue are uniformly dispersed, the filler composition for plastic surgery can be successfully administered by applying a small force to the syringe even when using a thin injection needle.

$$[\text{Equation 1}]$$

$$PSD = (Dv\,(90) - Dv\,(10))/Dv\,(50)$$

In Equation 1, Dv (10) is the size at which the biodegradable particle distribution is within 10% (the size of the particle at the 10% position based on the volume listed from the smallest particle diameter in a biodegradable particle distribution), Dv (50) is the size at which the biodegradable particle distribution is within 50% (the size of the particle at the 50% position based on the volume listed from the smallest particle diameter in a biodegradable particle distribution), and Dv (90) is the size at which the biodegradable particle distribution is within 90% (the size of the particle at the 90% position based on the volume listed from the smallest particle diameter in a biodegradable particle distribution).

Specifically, in Equation 1, Dv (10) may be 5 to 35 $\mu$m, 7 to 33 $\mu$m, 10 to 30 $\mu$m, 11 to 28 $\mu$m, 12 to 25 $\mu$m, or 12 to 22 $\mu$m, Dv (50) may be 10 to 50 $\mu$m, 13 to 47 $\mu$m, 15 to 45 $\mu$m, 17 to 43 $\mu$m, 19 to 42 $\mu$m, or 20 to 42 $\mu$m, and Dv (90) may be 20 to 90 $\mu$m, 25 to 85 $\mu$m, 27 to 82 $\mu$m, 29 to 80 $\mu$m, 30 to 78 $\mu$m, or 32 to 75 $\mu$m, but they are not limited thereto.

The biodegradable particles may be particles each having a network structure therein. Specifically, a three-dimensional network structure that is regular, irregular, or a combination thereof may be formed inside each of the biodegradable particles. As the network structure is present inside each of the biodegradable particles, the biodegradable particles can have high strength, which allows human tissue to be efficiently restored or replaced. In addition, as the biodegradable particles have high strength, the strength of the composite comprising them increases, thereby enhancing the handling convenience of the composite.

The biodegradable particles may comprise a commonly-known biodegradable polymer. Specifically, the biodegradable particles may comprise at least one selected from the group consisting of polylactic acid (PLA), polyglycolic acid (PGA), poly(D,L-lactic acid-co-glycolic acid) (PLGA), polycaprolactone (PCL), polyvalerolactone (PVL), polyhydroxybutyrate (PHB), and polyhydroxyvalerate (PBV), but it is not limited thereto. Preferably, the biodegradable particles may be polylactic acid (PLA) particles.

The biodegradable particles may have a weight average molecular weight of 50,000 to 400,000 g/mole, but it is not limited thereto. Specifically, the weight average molecular weight of the biodegradable particles may be 60,000 to 350,000 g/mole, 70,000 to 300,000 g/mole, 90,000 to 250,000 g/mole, 100,000 to 200,000 g/mole, 130,000 to 190,000 g/mole, or 150,000 to 180,000 g/mole. As the weight average molecular weight of the biodegradable particles is within the above range, processing them into a composite is readily carried out, and the dispersibility of the composite can be enhanced.

The biodegradable particles may have a tab density of 0.1 to 0.25 g/ml, but it is not limited thereto. Specifically, the tap density of the biodegradable particles may be 0.1 to 0.24 g/ml, 0.11 to 0.23 g/ml, 0.12 to 0.21 g/ml, 0.13 to 0.18 g/ml, or 0.13 to 0.17 g/ml. As the tap density of the biodegradable particles is within the above range, the biodegradable particles can be densely distributed within the composite, thereby increasing the strength of the composite and enhancing the dispersibility of the composite.

Water-soluble polymer

The water-soluble polymer contained in the composite according to the present invention functions as a carrier to transport biodegradable particles and a matrix function to disperse and fix the biodegradable particles.

The water-soluble polymer may comprise a polymer having commonly-known water-solubility properties. Specifically, the water-soluble polymer may comprise at least one selected from the group consisting of hyaluronic acid (HA), methylcellulose (MC), ethylcellulose (EC), carboxymethylcellulose (CMC), hydroxymethylcellulose (HMC), hydroxyethylcellulose (HEC), hydroxypropylcellulose (HPC), hydroxymethyl methacrylate (HEMA), polyvinyl alcohol (PVOH), polyvinylpyrrolidone (PVP), and starch, but it is not limited thereto. Preferably, the water-soluble polymer may be hyaluronic acid. Specifically, the water-soluble polymer may be non-crosslinked hyaluronic acid.

The hyaluronic acid may be high-molecule hyaluronic acids such as sodium hyaluronate crosspolymer and sodium hyaluronate; medium-molecule hyaluronic acids such as hydroxypropyltrimonium hyaluronate and sodium acetylated hyaluronate; low-molecular hyaluronic acids such as potassium hyaluronate, hydrolyzed hyaluronic acid, and hydrolyzed sodium hyaluronate; ultra-low-molecule hyaluronic acids such as hyaluronic acid; or a combination thereof.

**EP 4 541 384 A1**

The water-soluble polymer may have a weight average molecular weight of 1,000,000 to 4,000,000 g/mole, but it is not limited thereto. Specifically, the weight average molecular weight of the water-soluble polymer may be 1,200,000 to 4,000,000 g/mole, 1,300,000 to 4,000,000 g/mole, 1,500,000 to 4,000,000 g/mole, 1,800,000 to 4,000,000 g/mole, 2,000,000 to 4,000,000 g/mole, 2,100,000 to 3,800,000 g/mole, or 2,200,000 to 3,600,000 g/mole. As the weight average molecular weight of the water-soluble polymer is within the above range, processing it into a composite is readily carried out, and the dispersibility of the composite can be enhanced.

According to the present invention, the weight ratio of the biodegradable particles to the water-soluble polymer may be 40:60 to 95:5, but it is not limited thereto. Specifically, the weight ratio of the biodegradable particles and the water-soluble polymer contained in the composite may be 45:55 to 95:5, 45:55 to 90:10, 50:50 to 90:10, 55:45 to 85:15, 60:40 to 85:15, 65:35 to 85:15, 70:30 to 85:15, 75:25 to 85:15, 80:20 to 85:15, or 70:30 to 80:20. As the weight ratio is within the above range, the dispersibility of the composite is excellent, and the composite can be efficiently used as a material for a filler composition for plastic surgery.

Meanwhile, the composite according to the present invention may have a compressive strength of 0.02 to 1.5 MPa, but it is not limited thereto. Specifically, the compressive strength of the composite according to the present invention may be 0.025 to 1.3 MPa, 0.03 to 1.2 MPa, 0.033 to 1.0 MPa, 0.035 to 1.0 MPa, 0.035 to 0.8 MPa, 0.035 to 0.6 MPa, 0.036 to 0.5 MPa, 0.036 to 0.45 MPa, 0.036 to 0.43 MPa, 0.037 to 0.4 MPa, 0.037 to 0.39 MPa, 0.037 to 0.38 MPa, or 0.037 to 0.37 MPa.

In addition, the composite according to the present invention may have an apparent volume of 10 to 40 ml/g, but it is not limited thereto. Specifically, the apparent volume of the composite according to the present invention may be 10 to 35 ml/g, 10 to 32 ml/g, 10 to 30 ml/g, 12 to 29 ml/g, 14 to 29 ml/g, 15 to 28 ml/g, 15.5 to 28 ml/g, or 16 to 28 ml/g.

In addition, the composite according to the present invention may have a porosity of 90 to 97.5% by volume, but it is not limited thereto. Specifically, the porosity of the composite according to the present invention may be 90 to 97% by volume, 90 to 96% by volume, or 90 to 95% by volume. The porosity may refer to the volume of pores present in the composite out of the total volume of the composite.

As the composite according to the present invention has compressive strength, apparent density, and porosity within the above specific ranges, it may have excellent long-term storage, handling convenience, and dispersibility. In particular, since the composite according to the present invention has a compressive strength adjusted to the specific range while it comprises biodegradable particles with controlled particle size distribution, when the composite is dispersed in a solvent for preparing a filler composition for plastic surgery, it can be uniformly dispersed within a short period of time.

Specifically, the suspension time of the composite according to the present invention in an aqueous solvent may be 30 minutes or less, but it is not limited thereto. More specifically, the suspension time of the composite according to the present invention in an aqueous solvent may be 1 to 30 minutes, 5 to 30 minutes, 10 to 30 minutes, 10 to 29 minutes, 10 to 25 minutes, 10 to 20 minutes, or 12 to 19 minutes. Here, the aqueous solvent may specifically be water, distilled water, deionized water, ultrapure water, or the like, but it is not limited thereto.

The composite according to the invention may be in the form of a cake formulation with a fluffy texture. As the composite has a cake formulation, it can have excellent long-term storage and dispersibility in a solvent.

Specifically, the composite according to the present invention may have a cylindrical shape, but it is not limited thereto. In addition, the composite may have an average diameter of 1 to 5 cm, 1 to 3 cm, 1 to 2.5 cm, 1.2 to 2.2 cm, or 1.5 to 2.0 cm, and an average height of 0.2 to 5 cm, 0.3 to 3 cm, 0.5 to 3 cm, or 0.5 to 2.5 cm, but they are not limited thereto.

**Process for preparing a composite**

The present invention may provide a process for preparing the above composite. Specifically, the process for preparing a composite according to the present invention comprises (1) dissolving a biodegradable raw material in a first solvent to prepare a biodegradable solution; (2) spraying the biodegradable solution into a second solvent having a lower freezing point than that of the first solvent to form biodegradable particles; (3) sorting the biodegradable particles by size; (4) adding the biodegradable particles sorted by size to a water-soluble polymer solution to prepare a mixed solution; (5) filling the mixed solution in a container; and (6) freeze-drying the mixed solution filled in the container to form a composite.

Step (1) is to dissolve a biodegradable raw material in a first solvent to prepare a biodegradable solution. Specifically, step (1) may be carried out by adding the biodegradable raw material to a first solvent in which two or more organic solvents are mixed and stirring it.

Specifically, the biodegradable raw material may comprise at least one selected from the group consisting of polylactic acid (PLA), polyglycolic acid (PGA), poly(D,L-lactic acid-co-glycolic acid) (PLGA), polycaprolactone (PCL), polyvalerolactone (PVL), polyhydroxybutyrate (PHB), and polyhydroxyvalerate (PBV), but it is not limited thereto.

The biodegradable raw material may have a weight average molecular weight of 50,000 to 400,000 g/mole, but it is not limited thereto. Specifically, the weight average molecular weight of the biodegradable raw material may be 60,000 to 350,000 g/mole, 70,000 to 300,000 g/mole, 90,000 to 250,000 g/mole, 100,000 to 200,000 g/mole, 130,000 to 190,000 g/mole, or 150,000 to 180,000 g/mole.

The first solvent may specifically be at least two selected from the group consisting of dimethyl sulfoxide, diethyl sulfoxide, ethylene carbonate, propylene carbonate, dimethyl carbonate, N-methyl-2-pyrrolidone, N-ethyl-2-pyrrolidone, N-hydroxyethyl-2-pyrrolidone, N-cyclohexyl-2-pyrrolidone, N-methylformamide, N,N-dimethylformamide, N-ethylformamide, N,N-ethylformamide, N-methylacetamide, N,N-dimethylacetamide, N,N-diethylacetamide, isopropyl acetate, ethyl acetate, methyl acetate, dimethyl ketone, diethyl ketone, methyl ethyl ketone, isopropyl ketone, methyl propyl ketone, methyl butyl ketone, methyl isobutyl ketone, and tetrahydrofuran, but it is not limited thereto.

Specifically, the first solvent may be a solvent in which a first organic solvent and a second organic solvent are mixed at a weight ratio of 70:30 to 98:2, 75:25 to 98:2, 80:20 to 95:5, 85:15 to 95:5, or 85:15 to 90:10. More specifically, the first solvent may be a mixed solvent of dimethyl sulfoxide and ethylene carbonate, but it is not limited thereto.

Step (2) is to spray the biodegradable solution into a second solvent having a lower freezing point than that of the first solvent to form biodegradable particles each having a network structure therein. Specifically, step (2) may be carried out by spraying the biodegradable solution into a second solvent that does not mix with the first solvent to be separated in phase and has a freezing point that is lower than the freezing point of the first solvent by 5 to 150°C (specifically, 90 to 120°C).

The temperature of the second solvent during the spraying may be specifically -45 to 0°C, -40 to -5°C, -35 to -10°C, or -30 to -10°C, but it is not limited thereto. As the temperature of the second solvent is within the above range, biodegradable particles having a desired particle size distribution and a network structure therein can be well formed.

The second solvent may be at least one selected from the group consisting of pentane, hexane, heptane, octane, nonane, and decane, but it is not limited thereto.

The spraying speed of the biodegradable solution sprayed into the second solvent may be 1 to 20 ml/minute, 3 to 15 ml/minute, or 5 to 10 ml/minute, but it is not limited thereto.

Step (3) is to sort the biodegradable particles by size. Specifically, step (3) may be carried out by feeding the biodegradable particles into a particle sorter to select particles having the required size.

Specifically, the biodegradable particles may be charged to a particle sorter that uses buoyancy to sort particle sizes. In such an event, the criteria for sorting the particle size are not particularly limited, but the particle size may be sorted such that Dv (50) (average particle diameter) is 10 to 60 $\mu$m (specifically, 13 to 47 $\mu$m or 15 to 45 $\mu$m). As the above step is carried out, the particle size distribution of the biodegradable particles can be controlled to a specific range. As a composite is prepared using the biodegradable particles with a controlled particle size distribution, a composite with excellent dispersibility in a solvent can be prepared.

Step (4) is to add the biodegradable particles sorted by size to a water-soluble polymer solution to prepare a mixed solution. Specifically, step (4) may be carried out by adding the biodegradable particles sorted by size to a solution with a controlled concentration of the water-soluble polymer and stirring it.

The water-soluble polymer contained in the water-soluble polymer solution may specifically comprise at least one selected from the group consisting of hyaluronic acid (HA), methylcellulose (MC), ethylcellulose (EC), carboxymethylcellulose (CMC), hydroxymethylcellulose (HMC), hydroxyethylcellulose (HEC), hydroxypropylcellulose (HPC), hydroxymethyl methacrylate (HEMA), polyvinyl alcohol (PVOH), polyvinylpyrrolidone (PVP), and starch, but it is not limited thereto.

The water-soluble polymer may have a weight average molecular weight of 1,000,000 to 4,000,000 g/mole, but it is not limited thereto. Specifically, the weight average molecular weight of the water-soluble polymer may be 1,200,000 to 4,000,000 g/mole, 1,300,000 to 4,000,000 g/mole, 1,500,000 to 4,000,000 g/mole, 1,800,000 to 4,000,000 g/mole, 2,000,000 to 4,000,000 g/mole, 2,100,000 to 3,800,000 g/mole, or 2,200,000 to 3,600,000 g/mole.

The content of the water-soluble polymer contained in the water-soluble polymer solution may be 0.2 to 2% by weight, 0.4 to 1.5% by weight, 0.5 to 1.3% by weight, 0.5 to less than 1% by weight, or 0.6 to 0.95% by weight, based on the total weight of the water-soluble polymer solution, but it is not limited thereto. As the content of the water-soluble polymer is within the above range, the strength of the composite can be secured while the dispersibility of the composite in a solvent can be increased.

The solvent contained in the water-soluble polymer solution may be a commonly known aqueous solvent (e.g., water, distilled water, or the like).

Meanwhile, the water-soluble polymer solution and the biodegradable particles may be mixed such that the weight ratio (a:b) of the water-soluble polymer (a) contained in the water-soluble polymer solution to the biodegradable particles (b) is 40:60 to 95:5, 45:55 to 95:5, 45:55 to 90:10, 50:50 to 90:10, 55:45 to 85:15, 60:40 to 85:15, 65:35 to 85:15, 70:30 to 85:15, 75:25 to 85:15, 80:20 to 85:15, or 70:30 to 80:20.

Step (5) is to fill a container with the mixed solution. Specifically, step (5) may be carried out by filling the mixed solution in a specific container to form a composite having a cake formulation.

The container may specifically be a sealable glass bottle (e.g., a vial), but it is not limited thereto.

The amount of the mixed solution filled in the container may be 10 to 80% by volume, 15 to 70% by volume, 15 to 65% by volume, 20 to 60% by volume, or 25 to 55% by volume, based on the total volume of the container, but it is not limited thereto. As the filling amount of the mixed solution is within the above range, it is possible to form a composite in a cake formulation having the required compressive strength.

Step (6) is to freeze-dry the mixed solution filled in the container to form a composite. Specifically, step (6) may comprise (6-1) freezing the mixed solution at -60 to -10°C to obtain a frozen product; (6-2) first heating the frozen product to a temperature of -5 to 5°C for 1 to 3 hours under a vacuum atmosphere; (6-3) second heating the frozen product, first heated, to a temperature of 20 to 25°C for 8 to 15 hours in a vacuum atmosphere; and (6-4) drying the frozen product, second heated, at a temperature of 25°C or higher for 20 to 40 hours in a vacuum atmosphere.

The temperature for freezing the mixed solution in step (6-1) may be -60 to -10°C, -50 to -20°C, or -40 to -30°C, but it is not limited thereto. In addition, the time for freezing the mixed solution may be 60 to 240 minutes, 90 to 180 minutes, or 120 to 150 minutes, but it is not limited thereto.

Steps (6-2) to (6-4) are to dry (evaporate) the solvent present in the frozen product by gradually raising the temperature of the frozen product. In such an event, the final degree of vacuum in each step may be 0.1 to 30 mTorr, 0.5 to 20 mTorr, or 1 to 10 mTorr, but it is not limited thereto.

Specifically, step (6-2) may be carried out by first raising the temperature of the frozen product, which is -60 to -10°C, to a temperature of -5 to 5°C, -3 to 3°C, or -1 to 1°C over 1 to 3 hours or 1.5 to 2.5 hours in a vacuum atmosphere.

Step (6-3) may be carried out by second raising the first-raised temperature of the frozen product to a temperature of 20 to 25°C, 22 to 25°C, or 24 to 25°C over 8 to 15 hours or 9 to 11 hours in a vacuum atmosphere.

Step (6-4) may be carried out by raising the temperature of the second-raised frozen product to a temperature of 25°C or higher (specifically 25 to 30°C) over 20 to 40 hours or 22 to 30 hours in a vacuum atmosphere to finally dry it.

As the freeze-drying of the mixed solution is carried out through steps (6-1) to (6-4), a composite having a cake formulation can be efficiently formed.

Meanwhile, the process for preparing a composite according to the present invention may further comprise sterilizing the composite.

The sterilization may be carried out by gamma ray sterilization, e-beam sterilization, ethylene oxide sterilization, steam sterilization, or high-pressure steam sterilization, but it is not limited thereto. Specifically, the sterilization may be carried out using ethylene oxide at 30 to 40°C for 150 to 180 minutes. This procedure can further enhance the long-term storage of the composite.

The size of the biodegradable particles contained in the composite prepared through the above preparation process is not particularly limited, but the average particle diameter (Dv (50)) may be 15 to 60 $\mu$m. Specifically, the biodegradable particles may be first biodegradable particles having a Dv (50) particle size distribution of 15 to 30 $\mu$m, 17 to 29 $\mu$m, 18 to 28 $\mu$m, 19 to 27 $\mu$m, 20 to 25 $\mu$m, or 21 to 23 $\mu$m; second biodegradable particles having a Dv (50) particle size distribution of greater than 30 to 60 $\mu$m, greater than 30 to 55 $\mu$m, 35 to 50 $\mu$m, 35 to 48 $\mu$m, 38 to 45 $\mu$m, or 40 to 43 $\mu$m; or a mixture thereof, but they are not limited thereto.

Specifically, the biodegradable particles contained in the composite prepared through the above preparation process may have a particle size distribution (PSD) of 1.0 to 2.5, 1.0 to 2.3, 1.01 to 2.0, 1.02 to 1.9, 1.03 to 1.95, 1.04 to 1.9, 1.05 to 1.8, 1.05 to 1.6, 1.05 to 1.5, 1.08 to 1.4, 1.09 to 1.3, or 1.1 to 1.3, according to the following Equation 1, but it is not limited thereto.

[Equation 1]

$$PSD = (Dv\,(90) - Dv\,(10))/Dv\,(50)$$

In Equation 1, Dv (10) is the size at which the biodegradable particle distribution is within 10%, Dv (50) is the size at which the biodegradable particle distribution is within 50%, and Dv (90) is the size at which the biodegradable particle distribution is within 90%.

In the present invention, a composite is prepared by filling a mixed solution obtained by mixing biodegradable particles with a specific particle size distribution and a water-soluble polymer solution in a sealable container and freeze-drying the same; thus, it is possible to obtain a composite with a specific cake formulation and controlled compressive strength.

As the composite according to the present invention has a cake formulation while it comprises biodegradable particles with controlled particle size distribution, it has excellent long-term storage and dispersibility and enhanced handling convenience by virtue of controlled compressive strength. In addition, as the composite according to the present invention comprises biodegradable particles and a water-soluble polymer, it can have biocompatibility and biodegradability.

Accordingly, the composite according to the present invention can be advantageously used as a material to restore or replace human tissue. In addition, the composite according to the present invention can be used as a carrier for cells or drugs, a culture medium for cells, or the like.

Filler composition for plastic surgery

The present invention provides a filler composition for plastic surgery in which the above composite is dispersed. Specifically, the filler composition for plastic surgery according to the present invention may be one in which a composite having the same composition and characteristics as described above is dispersed in a solvent.

The filler composition for plastic surgery according to the present invention can be prepared within a short period of time (e.g., within 30 minutes) by virtue of the enhanced dispersibility of the composite. That is, as a sealed composite having a cake formulation in a solid phase is used, solubility and dispersibility are enhanced, making it possible to prepare a filler composition for plastic surgery within a short period of time. Thus, it is possible in the present invention to readily prepare a filler composition for plastic surgery immediately before the treatment. In addition, since the composite comprises biodegradable particles with a controlled particle size distribution, the filler composition for plastic surgery of the present invention obtained by dispersing it in a solvent allows the operator to easily perform the treatment while applying a small force to the syringe even when a needle with a thin diameter is used. Thus, the filler composition for plastic surgery according to the present invention can reduce operator discomfort and fatigue while improving the operating environment.

For example, the filler composition for plastic surgery according to the present invention (e.g., a composition in which a composite having an average particle diameter (Dv (50)) of 60 $\mu$m or less is dispersed) may have an injection force of 2.0 N or less for an injection needle having a gauge (G) of 26, specifically, 0.3 to 1.5 N, 0.5 to 1.3 N, 0.7 to 1.25 N, or 0.9 to 1.20 N, but it is not limited thereto. In addition, the filler composition for plastic surgery according to the present invention (e.g., a composition in which a composite having an average particle diameter (Dv (50)) of 33 $\mu$m or less is dispersed) may have an injection force of 3.0 N or less for an injection needle having a gauge (G) of 30, specifically, 1.6 to 2.6 N, 1.7 to 2.4 N, 1.8 to 2.2 N, or 1.9 to 2.0 N, but it is not limited thereto.

Mode for the Invention

Hereinafter, the present invention will be described in detail with reference to examples. But the scope of the present invention is not limited to the Examples.

Example 1

9 g of a polylactic acid (PLA) with a weight average molecular weight of 170,000 g/mole was dissolved in 150 ml of a mixed solvent of dimethyl sulfoxide and ethylene carbonate at a weight ratio of 90:10 to produce a polylactic acid solution.

Next, the polylactic acid solution thus prepared was sprayed into n-hexane cooled to -20°C or lower at a spray rate of 4.5 ml/minute and a spray air volume of 6 liters/minute to form frozen polylactic acid particles in n-hexane. The frozen polylactic acid particles thus formed were obtained, added into water at 1 to 3°C, and stirred to remove the mixed solvent (dimethyl sulfoxide and ethylene carbonate) contained in the frozen polylactic acid particles, thereby preparing polylactic acid particles (PLA particles).

Subsequently, the polylactic acid particles thus prepared were sorted using a particle sorter to obtain polylactic acid particles with a particle diameter of 60 $\mu$m or less (Dv (50): 41.9 $\mu$m).

Next, the polylactic acid particles with a particle diameter of 60 $\mu$m or less were added to a sodium hyaluronate solution having a concentration of 0.6% (HA solution = 99.4% by weight of distilled water + 0.6% by weight of sodium hyaluronate) such that the weight ratio of the polylactic acid particles to the sodium hyaluronate (HA) was 85:15, followed by mixing thereof to prepare a mixed solution.

Next, 5.20 g of the mixed solution (170 mg of PLA particles and 30 mg of HA) thus prepared was filled in a 10-ml vial.

Next, the mixed solution filled in the vial was frozen at -40 to -30°C to obtain a frozen product. The temperature of the frozen product thus obtained was first raised from -30°C to 0°C over 2 hours in a vacuum atmosphere, the temperature was then raised again from 0°C to 25°C over 10 hours, and it was then dried at 25°C for 24 hours to form a composite in the vial.

Thereafter, the vial containing the composite was sterilized using ethylene oxide (EO) gas, and residual moisture was removed through vacuum drying to prepare a composite with a diameter of 1.8 cm and a height of 2.2 cm in the vial.

Examples 2 to 4

Each composite was prepared through the same procedure as in Example 1, except that the concentration of sodium hyaluronate (HA) contained in the mixed solution filled in a vial, the amount of mixed solution filled, and the diameter and height of the composite were adjusted as shown in Table 1 below.

[Table 1]

| | PLA/HA concentration | Charged amount (g) of the mixed solution | Weight ratio of PLA:HA = 85:15 | | Composite diameter (cm) | Composite height (cm) |
|---|---|---|---|---|---|---|
| | | | PLA (mg) | HA (mg) | | |
| Ex. 1 | HA concentration: 0.6% PLA concentration: 3.4% | 5.20 | 170 | 30 | 1.8 | 2.2 |
| Ex. 2 | HA concentration: 0.8% PLA concentration: 4.5% | 3.95 | 170 | 30 | 1.8 | 1.5 |
| Ex. 3 | HA concentration: 1.0% PLA concentration: 5.7% | 3.20 | 170 | 30 | 1.8 | 1.2 |
| Ex. 4 | HA concentration: 1.2% PLA concentration: 6.8% | 2.70 | 170 | 30 | 1.8 | 0.8 |

**Example 5**

A composite with a diameter of 1.8 cm and a height of 0.55 cm was prepared through the same procedure as in Example 1, except that the polylactic acid particles were sorted using a particle sorter to obtain polylactic acid particles with a particle diameter of 33 $\mu$m or less (Dv (50): 21.1 $\mu$m), which were added to the sodium hyaluronate solution.

**Comparative Example 1**

A composite with a diameter of 1.8 cm was prepared through the same procedure as in Example 1, except that the mixed solution (170 mg of PLA particles and 30 mg of HA) was filled in a vial and was vacuum-dried at 25°C for 16 hours in a vacuum atmosphere (5 mTorr) (i.e., no freeze-drying and sterilization steps were performed).

**Comparative Example 2**

A composite with a diameter of 1.8 cm and a height of 2.2 cm was prepared through the same procedure as in Example 1, except that the polylactic acid particles were sorted using a particle sorter to obtain polylactic acid particles with a particle diameter of 300 $\mu$m or less (Dv (50): 41.7 $\mu$m), which were added to the sodium hyaluronate solution.

**Test Example 1: Measurement of particle size distribution**

The polylactic acid particles prepared in Examples 1 and 5 and Comparative Example 2 were each measured for particle size distribution according to the following measurement method. The results are shown in Table 2 below.

   * Method for measuring particle size distribution

   1) The composite were added to water and stirred for 30 minutes to prepare a suspension.
   2) The prepared suspension was analyzed with a particle size distribution meter (manufacturer: Malvern Instrument, model name: Mastersizer 3000-Maz6140) to measure the particle size distribution of the polylactic acid particles.
   3) The particle size distribution (PSD) was calculated from the values measured in step 2) using the following Equation 1.

$$[\text{Equation 1}]$$
$$PSD = (Dv\ (90) - Dv\ (10))/Dv\ (50)$$

In Equation 1, Dv (10) is the size at which the biodegradable particle distribution is within 10%, Dv (50) is the size at which the biodegradable particle distribution is within 50%, and Dv (90) is the size at which the biodegradable particle distribution is within 90%.

[Table 2]

|  | Dv (10) | Dv (50) | Dv (90) | Particle size distribution |
|---|---|---|---|---|
| Ex. 1 | 20.9 $\mu$m | 41.9 $\mu$m | 73.9 $\mu$m | 1.265 |
| Ex. 5 | 11.6 $\mu$m | 21.1 $\mu$m | 34.4 $\mu$m | 1.081 |
| C. Ex. 2 | 13.1 $\mu$m | 41.7 $\mu$m | 140 $\mu$m | 3.043 |

Referring to Table 2 above, the polylactic acid particles prepared in Examples 1 and 5 each had a particle size distribution in the range of 1.0 to 2.5. Here, as the particle size distribution of polylactic acid particles was within the above range, the present invention could increase the use convenience (utility) of the composite. That is, with a particle size distribution within the above range, the dispersibility of the composite is excellent, and the suspension is discharged smoothly even when a relatively small force (injection force) is applied to the syringe filled with the suspension in which the composite is dispersed. This can be confirmed in Test Examples 7 and 8 below.

Meanwhile, the polylactic acid particles prepared in Comparative Example 2 had a particle size distribution outside the range of 1.0 to 2.5.

**Test Example 2: Measurement of tap density**

The polylactic acid (PLA) particles obtained through the process of sorting with a particle sorter in Examples 1 and 5 were each measured for tap density according to the following measurement method. The results are shown in Table 3 below.

* Method for measuring tap density

1) The weight of a measuring cylinder was measured, and 20 to 25 ml of polylactic acid particles was placed in the measuring cylinder.

2) The weight of the measuring cylinder containing the polylactic acid particles was measured to calculate the weight of the polylactic acid particles only.

3) The measuring cylinder containing the polylactic acid particles was mounted on a tap density meter (manufacturer: Bettersize, model name: BeDensi T1 Pro).

4) The tap density meter was operated under the conditions of a tapping speed of 250 times/minute and a tapping count of 1,250 times.

5) Upon completion of the tapping, the measuring cylinder was taken out, and the volume of the polylactic acid particles was measured.

6) The weight of the polylactic acid particles measured in step 2) was divided by the volume of the polylactic acid particles measured in step 5) to calculate the tap density (to measure tap density for each of 5 samples and obtain the average value).

[Table 3]

| Sample | PLA particle size | Weight (g) | Volume (ml) | Tap density (g/ml) | Avg. tap density (g/ml) |
|---|---|---|---|---|---|
| 1 | 33 $\mu$m or less (Ex. 5) | 3.1 | 19.4 | 0.160 | 0.165 |
| 2 |  | 3.0 | 17.6 | 0.170 |  |
| 3 |  | 3.2 | 18.1 | 0.177 |  |
| 4 |  | 3.2 | 19.8 | 0.162 |  |
| 5 |  | 3.1 | 19.8 | 0.157 |  |
| 1 | 60 $\mu$m or less (Ex. 1) | 2.0 | 14.8 | 0.135 | 0.138 |
| 2 |  | 2.9 | 19.8 | 0.146 |  |
| 3 |  | 3.0 | 20.8 | 0.144 |  |
| 4 |  | 2.9 | 22.0 | 0.132 |  |
| 5 |  | 2.4 | 18.0 | 0.133 |  |

Referring to Table 3 above, the polylactic acid particles prepared in Examples 1 and 5 each had a tap density in the range of 0.1 to 0.2 g/ml. Here, in Example 5, where the particle size of the polylactic acid particles was smaller than that of Example 1, the tap density of the polylactic acid particles was larger. Thus, it is expected that bonding in high density within the composite would be achieved.

**Test Example 3: Measurement of compressive strength**

The composites prepared in Examples 1 to 4 and Comparative Examples 1 and 2 were each measured for compressive strength using an Instron 5848 (model name) instrument (measurement conditions - compression speed: 10 mm/minute, maximum compression ratio: 75%). The results are shown in Table 4 below.

[Table 4]

|  | HA concentration (%) | Compressive strength (MPa) |
|---|---|---|
| Ex. 1 | 0.6 | 0.03750 |
| Ex. 2 | 0.8 | 0.36316 |
| Ex. 3 | 1.0 | 0.50605 |
| Ex. 4 | 1.2 | 0.94671 |
| C. Ex. 1 | 0.6 | Not measurable (irregular shape) |
| C. Ex. 2 | 0.6 | 0.01998 |

Referring to Table 4 above, the composites in Examples 1 to 4 each had a compressive strength in the range of 0.03 to 1 MPa. Further, it is understood that the concentration of sodium hyaluronate (HA) has an impact on the compressive strength of the composite.

Meanwhile, the composite of Comparative Example 2 had a lower compressive strength than those of the composites of Examples 1 to 4. In addition, since the composite of Comparative Example 1 had an irregular shape, the compressive strength could not be measured.

**Test Example 4: Measurement of apparent volume**

The composites prepared in Examples 1 to 4 and Comparative Example 1 were each measured for apparent volume using the diameter and height of the composite. The results are shown in Table 5 below.

[Table 5]

|  | HA concentration (%) | Apparent volume (ml/g) |
|---|---|---|
| Ex. 1 | 0.6 | 27.9 |
| Ex. 2 | 0.8 | 17.0 |
| Ex. 3 | 1.0 | 15.2 |
| Ex. 4 | 1.2 | 10.1 |
| C. Ex. 1 | 0.6 | Not measurable (irregular shape) |

Referring to Table 5 above, the composites in Examples 1 to 4 each had an apparent volume in the range of 10 to 40 ml/g. Further, it is understood that in order to increase the porosity of the composite, it is desirable to lower the concentration of sodium hyaluronate (HA). Here, if the composite has high porosity, it can be suspended rapidly in an aqueous solvent for use, thereby increasing the use convenience of the composite. This can be confirmed in Test Examples 7 and 8 below.

Meanwhile, since the composite of Comparative Example 1 had an irregular shape, the apparent volume could not be measured.

**Test Example 5: Observation of formulation**

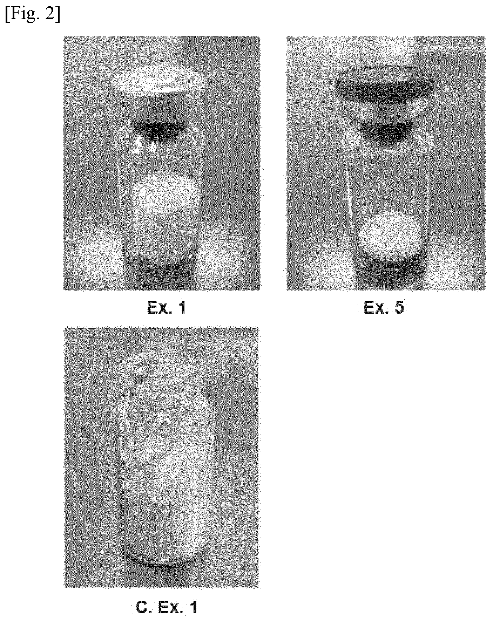

The formulations of the composites prepared in Examples 1, 5, and Comparative Example 1 were each visually observed. The results are shown in Fig. 2.

Referring to Fig. 2, the composites of Examples 1 and 5 each had a cake formulation (cylindrical shape). In contrast, the composite of Comparative Example 1 did not have a regular shape since the mixed solution swelled during the vacuum drying process.

Here, as the composites in Examples 1 and 5 each had a cake formulation, the present invention can increase the dispersibility and use convenience of the composite. This can be confirmed in Test Examples 7 and 8 below.

**Test Example 6: Observation of the internal structure of polylactic acid particles and the structure of composite**

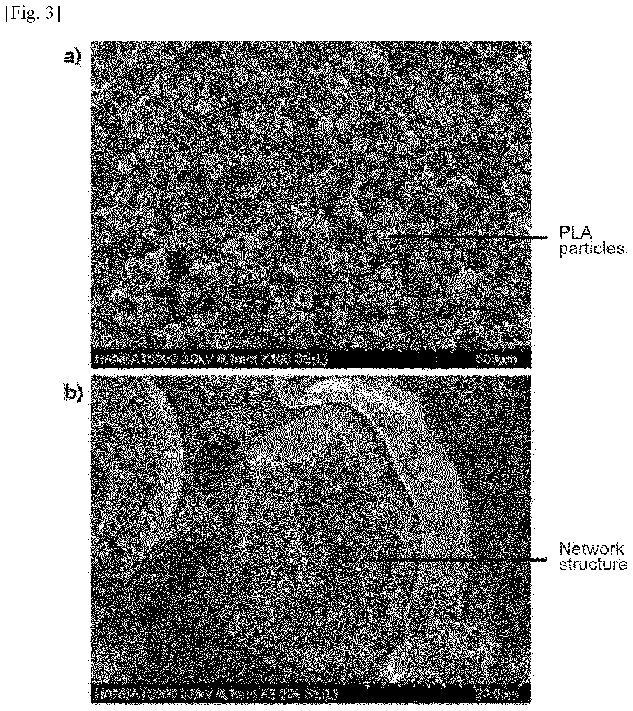

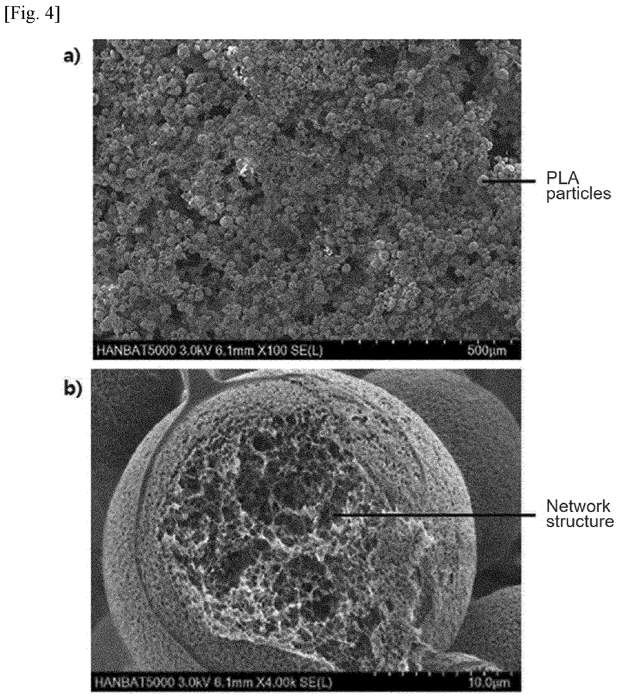

The composites prepared in Examples 1 and 5 were each cut in the vertical direction, and the cross-section was observed using a scanning electron microscope (manufacturer: Hitachi High Technology, model name: Hitachi su5000). The results are shown in Figs. 3 and 4.

Referring to a) each shown in Figs. 3 and 4, in the composites of Examples 1 and 5, polylactic acid particles each maintaining its shape were uniformly present inside the composite. In addition, referring to b) each shown in Figs. 3 and 4, the polylactic acid particles present in the composites of Examples 1 and 5 each had a network structure therein.

**Test Example 7: Measurement of suspension time**

The composites prepared in Examples 1 to 4 and Comparative Example 1 were each measured for suspension time according to the following measurement method. The results are shown in Table 6 below.

* Method for measuring suspension time

1) 8 ml of distilled water was added to a vial containing the composite, which was left for about 5 minutes.

2) The vial was stirred for 5 minutes with the rpm of a vortex mixer set to 3,000.

3) Whether the composite in the vial had been completely dissolved and granulated was visually checked.

4) The procedure in step 2) was repeated until the composite was completely dissolved, and the time taken for being granulated was measured.

[Table 6]

|  | HA concentration (%) | Sample | rpm | Suspension time (min) | Avg. suspension time (min) |
|---|---|---|---|---|---|
| Ex. 1 | 0.6 | 1 | 3,000 | 15 | 15.0 |
|  |  | 2 |  | 15 |  |
|  |  | 3 |  | 15 |  |
| Ex. 2 | 0.8 | 1 | 3,000 | 20 | 18.3 |
|  |  | 2 |  | 15 |  |
|  |  | 3 |  | 20 |  |
| Ex. 3 | 1.0 | 1 | 3,000 | 25 | 26.6 |
|  |  | 2 |  | 25 |  |
|  |  | 3 |  | 30 |  |
| Ex. 4 | 1.2 | 1 | 3,000 | 25 | 28.3 |
|  |  | 2 |  | 30 |  |
|  |  | 3 |  | 30 |  |

Referring to Table 6 above, the composites in Examples 1 to 4 were each rapidly suspended (dispersed) with a suspension time of 30 minutes or less. As the suspension is accomplished in a short period of time, the composite according to the present invention has excellent use convenience. For example, when the composite is suspended in an aqueous solvent at the treatment site for skin treatment, it can be suspended within a short period of time, increasing treatment efficiency.

Here, the suspension of the composite according to the present invention is possible in a short period of time since the composite has a cake formulation and has particle size distribution, compressive strength, and apparent volume within specific ranges. This supports the importance of controlling the shape, compressive strength, apparent volume, and particle size distribution of polylactic acid particles of the composite.

Further, it was confirmed that the lower the concentration of sodium hyaluronate (HA), the shorter the suspension time. Thus, in order to increase the use convenience of the composite, it is desirable to control the concentration of hyaluronic acid, which forms the support structure (frame) of the composite.

**Test Example 8: Measurement of injection force**

The composites prepared in Examples 1 and 5 and Comparative Example 2 were each measured for injection force according to the following measurement method. The results are shown in Table 7 below.
* Method for measuring injection force
1) 5 ml of distilled water was added to a vial containing the composite and stirred with a vortex mixer until the composite was dissolved and granulated (suspension preparation).
2) 0.5 to 0.6 ml of the suspension in the vial was charged to a 1-ml disposable syringe.
3) A 26 G or 30 G injection needle was assembled to the syringe, and the syringe was fixed into the support of a universal testing machine (manufacturer: TestOne, model name: TO-102) with the needle facing downward.
4) The universal testing machine was operated to press the push bar attached to the injection tube at 1 mm/sec until the injection tube was completely empty, and the measured force was recorded.
5) In the graph recording the measured force, calculate the average value (input force) of the forces measured at a point 5 mm to the right of the starting point, at a point 5 mm to the left of the ending point, and the midpoint between these two points.

[Table 7]

|  | Needle gauge (G) | Injection force (N) |
| --- | --- | --- |
| Ex. 1 | 26 | 1.12 |
| Ex. 5 | 26 | 0.80 |
|  | 30 | 1.80 |
| C. Ex. 2 | 26 | Not measurable |
|  | 30 | Not measurable |

Referring to Table 7 above, the composites of Examples 1 and 5 each had a low injection force; thus, the suspension in which the composite was dispersed was smoothly discharged from the syringe even when a relatively small force was applied. As the injection force is small, the composite according to the present invention has excellent use convenience. For example, when a suspension in which the composite is dispersed is used at the treatment site, the suspension is smoothly discharged from the syringe and injected into the skin even if a relatively small force is applied to the syringe. Thus, the convenience of the treatment can be enhanced.
Meanwhile, the composite of Comparative Example 2 had a very large particle size when dispersed. Thus, the suspension in which the composite of Comparative Example 2 was dispersed could not pass through the injection needle to block it, whereby it was impossible to measure the injection force. Accordingly, it is understood that the suspension in which the composite of Comparative Example 2 is dispersed can be hardly used in the treatment site.
This result supports the importance of controlling the shape, compressive strength, apparent volume, and particle size distribution of polylactic acid particles of the composite.

**Claims**

1. A composite, which comprises biodegradable particles; and a water-soluble polymer, wherein the particle size distribution (PSD) of the biodegradable particles according to the following Equation 1 is 1.0 to 2.5:

$$[\text{Equation 1}]$$

$$PSD = (Dv\,(90) - Dv\,(10))/Dv\,(50)$$

   in Equation 1, Dv (10) is the size at which the biodegradable particle distribution is within 10%, Dv (50) is the size at which the biodegradable particle distribution is within 50%, and Dv (90) is the size at which the biodegradable particle distribution is within 90%.

2. The composite of claim 1, wherein the composite has a compressive strength of 0.02 to 1.5 MPa.

3. The composite of claim 1, wherein, in Equation 1, Dv (10) is 5 to 35 $\mu$m, and Dv (90) is 20 to 90 $\mu$m.

4. The composite of claim 1, wherein the composite has an apparent volume of 10 to 40 ml/g.

5. The composite of claim 1, wherein the biodegradable particles each have a network structure therein.

6. The composite of claim 1, wherein the biodegradable particles comprise at least one selected from the group consisting of polylactic acid (PLA), polyglycolic acid (PGA), poly(D,L-lactic acid-co-glycolic acid) (PLGA), polycaprolactone (PCL), polyvalerolactone (PVL), polyhydroxybutyrate (PHB), and polyhydroxyvalerate (PBV).

7. The composite of claim 1, wherein the water-soluble polymer comprises at least one selected from the group consisting of hyaluronic acid (HA), methylcellulose (MC), ethylcellulose (EC), carboxymethylcellulose (CMC), hydroxymethylcellulose (HMC), hydroxyethylcellulose (HEC), hydroxypropylcellulose (HPC), hydroxymethyl methacrylate (HEMA), polyvinyl alcohol (PVOH), polyvinylpyrrolidone (PVP), and starch.

8. The composite of claim 1, wherein the suspension time of the composite in an aqueous solvent is 30 minutes or less.

9. A process for preparing a composite, which comprises:

   (1) dissolving a biodegradable raw material in a first solvent to prepare a biodegradable solution;
   (2) spraying the biodegradable solution into a second solvent having a lower freezing point than that of the first solvent to form biodegradable particles;
   (3) sorting the biodegradable particles by size;
   (4) adding the biodegradable particles sorted by size to a water-soluble polymer solution to prepare a mixed solution;
   (5) filling the mixed solution in a container; and
   (6) freeze-drying the mixed solution filled in the container to form a composite,
   wherein the particle size distribution (PSD) of the biodegradable particles contained in the composite according to the following Equation 1 is 1.0 to 2.5:

$$[\text{Equation 1}]$$

$$PSD = (Dv\,(90) - Dv\,(10))/Dv\,(50)$$

   in Equation 1, Dv (10) is the size at which the biodegradable particle distribution is within 10%, Dv (50) is the size at which the biodegradable particle distribution is within 50%, and Dv (90) is the size at which the biodegradable particle distribution is within 90%.

10. The process for preparing a composite according to claim 9, wherein the freeze-drying in step (6) comprises:

    (6-1) freezing the mixed solution at -60 to -10°C to obtain a frozen product;
    (6-2) first heating the frozen product to a temperature of -5 to 5°C for 1 to 3 hours under a vacuum atmosphere;
    (6-3) second heating the frozen product, first heated, to a temperature of 20 to 25°C for 8 to 15 hours in a vacuum atmosphere; and
    (6-4) drying the frozen product, second heated, at a temperature of 25°C or higher for 20 to 40 hours in a vacuum atmosphere.

11. The process for preparing a composite according to claim 9, wherein, in step (4), the content of the water-soluble polymer contained in the water-soluble polymer solution is 0.2 to 2% by weight based on the total weight of the water-soluble polymer solution.

12. A filler composition for plastic surgery in which the composite according to any one of claims 1 to 8 is dispersed.

13. The filler composition for plastic surgery of claim 12, wherein the filler composition for plastic surgery has an injection force of 2.0 N or less for an injection needle having a gauge (G) of 26.

[Fig. 1]

```
                    ┌──────────────┐
                    │    Start     │
                    └──────┬───────┘
                           ▼
          ┌────────────────────────────────┐
          │  Preparation of a biodegradable │ ⟋ S (1)
          │            solution             │
          └────────────────┬───────────────┘
                           ▼
          ┌────────────────────────────────┐
          │   Formation of biodegradable    │ ⟋ S (2)
          │           particles             │
          └────────────────┬───────────────┘
                           ▼
          ┌────────────────────────────────┐
          │    Sorting the biodegradable    │ ⟋ S (3)
          │        particles by size        │
          └────────────────┬───────────────┘
                           ▼
          ┌────────────────────────────────┐
          │   Preparation of a mixed solution │ ⟋ S (4)
          └────────────────┬───────────────┘
                           ▼
          ┌────────────────────────────────┐
          │     Filling the mixed solution   │ ⟋ S (5)
          └────────────────┬───────────────┘
                           ▼
          ┌────────────────────────────────┐
          │         Freeze-drying            │ ⟋ S (6)
          └────────────────┬───────────────┘
                           ▼
                    ┌──────────────┐
                    │     End      │
                    └──────────────┘
```

[Fig. 2]

Ex. 1

Ex. 5

C. Ex. 1

[Fig. 3]

a) HANBAT5000 3.0kV 6.1mm X100 SE(L)    500μm — PLA particles

b) HANBAT5000 3.0kV 6.1mm X2.20k SE(L)    20.0μm — Network structure

[Fig. 4]

a)

HANBAT5000 3.0kV 6.1mm X100 SE(L)     500μm

— PLA particles

b)

HANBAT5000 3.0kV 6.1mm X4.00k SE(L)     10.0μm

— Network structure

## INTERNATIONAL SEARCH REPORT

| International application No. |
|---|
| **PCT/KR2023/008196** |

### A. CLASSIFICATION OF SUBJECT MATTER

**A61L 27/48**(2006.01)i; **A61L 27/26**(2006.01)i; **A61L 27/18**(2006.01)i; **A61L 27/20**(2006.01)i; **A61L 27/36**(2006.01)i; **A61L 27/60**(2006.01)i

According to International Patent Classification (IPC) or to both national classification and IPC

### B. FIELDS SEARCHED

Minimum documentation searched (classification system followed by classification symbols)

A61L 27/48(2006.01); A61F 2/00(2006.01); A61L 27/14(2006.01); A61L 27/26(2006.01); A61L 27/56(2006.01); C08J 3/12(2006.01); C08L 67/04(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 생분해성 (biodegradable), 입자 (particle), 수용성 고분자 (water soluble polymer), 복합체 (complex), 입도분포 (particle size distribution), 필러 (filler), 분산 (dispersion)

### C. DOCUMENTS CONSIDERED TO BE RELEVANT

| Category* | Citation of document, with indication, where appropriate, of the relevant passages | Relevant to claim No. |
|---|---|---|
| X | KR 10-2266385 B1 (ULTRA V CO., LTD.) 21 June 2021 (2021-06-21)<br>See paragraphs [0062]-[0086], [0129], [0201]-[0211] and [0218]; claims 1, 6, 10-14 and 18; figures 4-5; and table 4. | 1-4,6-8,12-13 |
| Y | | 5,9-11 |
| Y | KR 10-1725279 B1 (VAIM CO., LTD.) 10 April 2017 (2017-04-10)<br>See paragraphs [0011], [0023], [0037] and [0075]-[0090]; claims 1 and 3; and figures 2 and 6. | 5,9-11 |
| A | KR 10-2266384 B1 (ULTRA V CO., LTD.) 21 June 2021 (2021-06-21)<br>See entire document. | 1-13 |

☑ Further documents are listed in the continuation of Box C.  ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **11 September 2023** | **12 September 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

<table>
<tr><td colspan="2" align="center">**INTERNATIONAL SEARCH REPORT**</td><td>International application No.<br>**PCT/KR2023/008196**</td></tr>
</table>

**C.    DOCUMENTS CONSIDERED TO BE RELEVANT**

| Category* | Citation of document, with indication, where appropriate, of the relevant passages | Relevant to claim No. |
|---|---|---|
| A | (주)바임. 중소기업 기술개발사업 최종보고서. PLA 마이크로파티클 제조공정 개선 연구. 09 September 2015, pp. 1-35, non-official translation (VAIM CO., LTD. Final Report on Small and Medium Business Technology Development Project. Research on Improving the PLA Microparticle Manufacturing Process.)<br>　　　See entire document. | 1-13 |
| A | KR 10-1929661 B1 (SONG, Suk Jin) 17 December 2018 (2018-12-17)<br>　　　See entire document. | 1-13 |
| A | KR 10-2019-0132113 A (SAMYANG BIOPHARMACEUTICALS CORPORATION) 27 November 2019 (2019-11-27)<br>　　　See entire document. | 1-13 |

Form PCT/ISA/210 (second sheet) (July 2022)

<table>
<tr><td colspan="2" align="center">**INTERNATIONAL SEARCH REPORT**<br>**Information on patent family members**</td><td colspan="2">International application No.<br><br>**PCT/KR2023/008196**</td></tr>
<tr><td align="center">Patent document<br>cited in search report</td><td align="center">Publication date<br>(day/month/year)</td><td align="center">Patent family member(s)</td><td align="center">Publication date<br>(day/month/year)</td></tr>
<tr><td>KR    10-2266385  B1</td><td>21 June 2021</td><td>EP      4032941   A1</td><td>27 July 2022</td></tr>
<tr><td></td><td></td><td>JP    2022-113151   A</td><td>03 August 2022</td></tr>
<tr><td></td><td></td><td>JP      7256304   B2</td><td>11 April 2023</td></tr>
<tr><td></td><td></td><td>US     11597834   B2</td><td>07 March 2023</td></tr>
<tr><td></td><td></td><td>US  2022-0235221   A1</td><td>28 July 2022</td></tr>
<tr><td>KR    10-1725279  B1</td><td>10 April 2017</td><td>BR  112018008405  A2</td><td>30 October 2018</td></tr>
<tr><td></td><td></td><td>BR  112018008405  B1</td><td>25 January 2022</td></tr>
<tr><td></td><td></td><td>CN    108350180   A</td><td>31 July 2018</td></tr>
<tr><td></td><td></td><td>CN    108350180   B</td><td>01 January 2021</td></tr>
<tr><td></td><td></td><td>WO  2017-073963   A1</td><td>04 May 2017</td></tr>
<tr><td></td><td></td><td>WO  2017-073963   A9</td><td>13 September 2018</td></tr>
<tr><td>KR    10-2266384  B1</td><td>21 June 2021</td><td>EP      4032561   A1</td><td>27 July 2022</td></tr>
<tr><td></td><td></td><td>JP    2022-113662   A</td><td>04 August 2022</td></tr>
<tr><td></td><td></td><td>JP      7312865   B2</td><td>21 July 2023</td></tr>
<tr><td></td><td></td><td>US  2022-0233744   A1</td><td>28 July 2022</td></tr>
<tr><td>KR    10-1929661  B1</td><td>17 December 2018</td><td>KR  10-2017-0126416   A</td><td>17 November 2017</td></tr>
<tr><td></td><td></td><td>WO  2017-196051   A1</td><td>16 November 2017</td></tr>
<tr><td>KR  10-2019-0132113  A</td><td>27 November 2019</td><td align="center">None</td><td></td></tr>
</table>

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 20170123099